# EUROPÄISCHE PATENTANMELDUNG

(11) **EP 2 305 318 A1**
(43) Veröffentlichungstag der Anmeldung: **06.04.2011**
(21) Anmeldenummer: 09075446.6
(22) Anmeldetag: 25.09.2009
(51) Int. Cl.: A61L 2/26

(54) **Integration eines Thermoschalters in einen Verschluss eines Sterilgutbehälters**

(71) Anmelder: Universität Potsdam, Institut für Biochemie und Biologie, 14476 Potsdam-Golm (DE)
(72) Erfinder: Sellrie, Dr. Frank, 14476 Potsdam (DE); Egbert, Bjoern, 16244 Schorfheide (DE)
(74) Vertreter: Boeckh, Tobias

(57) **Zusammenfassung**

Die Erfindung betrifft einen Verschluss eines Behälters für Sterilgut, ein Verfahren zum Verschließen eines Verschlusses und eine Verwendung eines Thermoschalters zum Verschließen eines Verschlusses eines Sterilgutbehälters.

## Beschreibung

Die Erfindung betrifft einen Verschluss eines Behälters für Sterilgut, ein Verfahren zum Verschließen eines Verschlusses und eine Verwendung eines Thermoschalters zum Verschließen eines Verschlusses eines Sterilgutbehälters.

Für eine Vielzahl biologischer und immuntechnologischer Anwendungen ist die Bereitstellung steriler Arbeitsmaterialien notwendig. Eine Methode diese Sterilität sicherzustellen ist das Autoklavieren, bzw. das Sterilisieren. Hierbei wird das zu autoklavierende Gut in einen gasdicht verschließbaren Druckbehälter eingeführt, der für die thermische Behandlung von Stoffen im Überdruckbereich eingesetzt wird. Durch das Autoklavieren, beziehungsweise die durch das Autoklavieren erfolgende Sterilisation werden die in den Autoklav eingeführten Materialien oder Gegenstände von lebenden Mikroorganismen sowie von Dauerformen dieser wie Sporen befreit. Es können Dampfsterilisationen, Heißluftsterilisationen oder fraktionierte Sterilisationen unterschieden werden. Durch die Heißluftsterilisation wird ein Gegenstand oder ein Behälter mittels heißer Luft auf eine Temperatur von etwa 120 bis zu 400°C temperiert. Die Dampfsterilisation ist das Standardverfahren in den meisten Laboren und Krankenhäusern, bei der das zu sterilisierende Gut etwa 20 Min auf 120°C bei 2 bar Druck im Wasserdampf erhitzt wird. Hierbei kann die Temperatur sowie der Druck an das zu sterilisierende Gut und den zu erreichenden Sterilisationsgrad angepasst werden. Bei der fraktionierten Sterilisation wird das sterile Gut an mehreren aufeinander folgenden Tagen jeweils auf etwa 100°C erhitzt und dazwischen bei Raumtemperatur gelagert. Hierdurch soll erreicht werden, dass besonders hitzeresistente Stadien von Mikroorganismen (zum Beispiel Bakteriensporen) während der Lagerung bei Raumtemperatur auskeimen und an den jeweils folgenden Tagen in denen das Gut auf 100°C erhitzt wird, abgetötet werden.

Im täglichen Laboralltag werden weitere Funktionskontrollen oder Nachweisverfahren durchgeführt, die dem Nachweis der Sterilisationsleistung zukommen. Für die laufende Kontrolle werden üblicherweise chemische Indikatorfelder auf den Verpackungen oder auf Papierklebestreifen verwendet, die bei den definierten Sterilisationsbedingungen einen Farbumschlag anzeigen. Die Papierklebestreifen sind als sogenanntes Autoklavierband oder Indikatorband, Sterilindikator oder Sterilisationsindikator bekannt. Hierbei sind Indikatoren auf die Oberfläche aufgetragen, die sich unter den Bedingungen der Sterilisation (Dampf-, beziehungsweise Heißluftsterilisation) verändern. Die Indikatoren sind als Etiketten erhältlich oder können als Klebeband auf Beutel, Flaschen oder Verpackungen aufgebracht werden. Durch den Farbumschlag der Indikatoren kann bestimmt werden, ob das zu sterilisierende Gut auf die vorgeschriebene Temperatur erhitzt wurde und so der Farbumschlag ausgelöst worden ist. Es ist jedoch nicht erkennbar ob die Vorrichtung, auf die der Klebestreifen aufgebracht wurde (beispielsweise eine Flasche oder ein Behälter) nach der Sterilisation bereits geöffnet wurde. Außerdem wird durch die Temperatureinwirkung auf das Klebeband die Verbindung zwischen dem Klebeband und dem zu sterilisierenden Gut intensiviert, wodurch wiederum das Klebeband nur schwer von dem Gut entfernt werden kann, was eine Wiederverwendbarkeit des Gutes erschwert. Das Klebeband an sich ist ein Einwegartikel und kann nicht wiederverwendet werden.

Ausgehend von diesem Stand der Technik ist es die Aufgabe der vorliegenden Erfindung eine Vorrichtung bereitzustellen, die eine Beurteilung darüber ermöglicht, ob eine Sterilisation stattgefunden hat und ob das Behältnis, welches das zu sterilisierende Gut enthält, bereits nach der Sterilisation geöffnet wurde.

Überraschenderweise wird diese Aufgabe durch die Merkmale der unabhängigen Ansprüche gelöst. Bevorzugte Ausführungsformen der Erfindung ergeben sich aus den Unteransprüchen.

Es war völlig überraschend, dass das erfindungsgemäße Problem mit einem Verschluss eines Behälters für Sterilgut der mindestens zwei Positionen (offen/geschlossen) einnehmen kann und eine Verschlusswippe, ein innenseitiges wiedereinrückbares Stellelement und einen in einer Halterung ortsfest befestigten Thermoschalter umfasst, gelöst werden kann. In einer ersten Position (offen) ist der Thermoschalter und das wiedereinrückbare innenseitige Stellelement derart zueinander angeordnet, dass eine Verformung des Thermoschalters eine Bewegung des wiedereinrückbaren innenseitigen Stellelementes in eine zweite Position (geschlossen) bewirkt. In der zweiten Position liegt das wiedereinrückbare innenseitige Stellelement an der Verschlusswippe an und drückt diese in einen Anschlag, wodurch die Verschlusswippe nur durch einen wesentlichen Kraftaufwand von außen eindrückbar ist. Der Behälter ist so geschlossen. Der in einen Deckel eines Behälters integrierte Verschluss kann an unterschiedliche Behälter, beziehungsweise Deckel angepasst werden, wobei auch die Größe und Form des Deckels variabel sind.

Wenn der Behälter für Sterilgut und der in einen Deckel integrierte Verschluss in einen Sterilisator oder Autoklav eingeführt werden, befindet sich der Verschluss vorteilhafterweise in einer offenen Position. In der offenen Position ist die Verschlusswippe eindrückbar, das heißt die Verschlusswippe kann von außen durch einen geringen Kraftaufwand eingedrückt werden und somit der Deckel des Behälters geöffnet werden. Das innenseitige wiedereinrückbare Stellelement liegt in der offenen Position, das heißt in der ersten Position nicht an der Verschlusswippe an und diese ist nicht in ihren Anschlag gedrückt. Im Sinne der Erfindung bezeichnet ein Anschlag, eine mechanische Position, bis zu der sich die Verschlusswippe bewegen lässt. Es kann jedoch auch bevorzugt sein, dass die Verschlusswippe nicht von dem Stellelement in ihren Anschlag gedrückt wird. Hierbei besteht kein Kontakt zwischen dem Stellelement und der Verschlusswippe, sondern das Stellelement ist derart in der zweiten Position arretiert, dass die Verschlusswippe nur begrenzt bewegbar ist. Durch einen wesentlichen Kraftaufwand, der von außen auf die Verschlusswippe ausgeübt wird, wird das Stellelement von der Verschlusswippe in die erste Position bewegt.

Während der Erhitzung des Behälters und folglich des Verschlusses, kommt es zu einer Verformung des Thermoschalters, der in eine Halterung ortsfest befestigt ist und in der ersten Position derart angeordnet ist, dass durch die Verformung des Thermoschalters eine Bewegung des wiedereinrückbaren innenseitigen Stellelements bewirkt wird. Die Bewegung des Thermoschalters erfolgt vorteilhafterweise durch die erfindungsgemäße Anordnung des Schalters in einer Ebene, das heißt in einer räumlichen Dimension. Im Sinne der Erfindung bezeichnet eine Verformung eine Änderung seiner Form infolge der Einwirkung einer äußeren Temperatur. Die Verformung kann als Längenänderung oder als Winkeländerung in Erscheinung treten und ist reversible, das heißt umkehrbar oder nicht dauerhaft.

Vorteilhafterweise befindet sich in der ersten Position das Stellelement in einer räumlichen Nähe zu dem Thermoschalter, so dass bei einer Erwärmung über eine definierte Temperatur eine Verformung des Thermoschalters erfolgt und durch diese Verformung ein Druck auf das Stellelement ausgeübt wird. Vorteilhafterweise entspricht die definierte Temperatur der zu erreichenden Sterilisationstemperatur. Der Verschluss kann einfach und schnell, beispielsweise durch den Austausch des Thermoschalters an eine zu erreichenden Sterilisationstemperatur, angepasst werden, wodurch eine Fehlfunktion des Thermoschalters, beziehungsweise des Verschlusses reduziert wird. Es kann jedoch auch vorteilhaft sein, wenn die Temperatur, bei der sich der Thermoschalter verformt nicht der Sterilisationstemperatur entspricht. Beispielsweise kann diese Temperatur über der Temperatur liegen, bei der eine Verformung des Thermoschalters erfolgt.

Im Sinne der Erfindung ist ein Thermoschalter oder Temperaturschalter ein Schalter, dessen Schaltzustand sich in Abhängigkeit von der Temperatur und von der materialspezifischen Temperaturabhängigkeit des Schalters ändert. Die Verformung wirkt auf das vorteilhafterweise in räumlicher Nähe zu dem Thermoschalter angeordneten Stellelement. Der durch die Verformung ausgelöste Druck bewirkt eine Bewegung des wiedereinrückbaren innenseitigen Stellelements, wodurch sich das Stellelement aus der ersten Position in die zweite Position bewegt. In dieser zweiten Position liegt das innenseitige Stellelement an der Verschlusswippe an und durch den von dem Stellelement auf die Verschlusswippe wirkenden Druck wird die Verschlusswippe in einen Anschlag gedrückt. In dieser Position ist die Verschlusswippe nicht bewegbar, das heißt der Verschluss kann von außen durch Druck der Verschlusswippe nicht ohne wesentlichen Kraftaufwand geöffnet werden. Somit wird während der Sterilisation durch eine Verformung des Thermoschalters, der Verschluss in eine geschlossene zweite Position gebracht und die Verschlusswippe durch das Stellelement blockiert.

Der Behälter ist nach der Sterilisation geschlossen und eine Person, die gegebenenfalls das in dem Behälter befindliche Sterilgut verwenden möchte, muss einen wesentlichen Kraftaufwand anwenden, um die Verschlusswippe einzudrücken. Vorteilhafterweise ist die Verschlusswippe in einem offenen Zustand mit einem geringen Kraftaufwand einzudrücken. Vorteilhafterweise kann der Deckel in einem offenen Zustand einfach bewegt werden, wobei dies in einem geschlossenen Zustand nicht ohne wesentlichen Kraftaufwand möglich ist. Hierdurch kann die Benutzung von nicht sterilisiertem Sterilgut oder von erneut kontaminiertem Sterilgut auf eine einfache und kostengünstige Weise verhindert werden. Nach Abkühlung des Behälters, beziehungsweise des Verschlusses und folglich des Thermoschalters wird die reversible Verformung des Thermoschalters rückgängig gemacht und der Thermoschalter kehrt in seine ursprünglich Form zurück, die in der ersten Position (offen) vorlag. Die Rückkehr der Verformung in die ursprüngliche Form des Thermoschalters kann durch das Material, beziehungsweise durch materialspezifische Faktoren beeinflusst werden, so dass dies bei einer definierten Temperatur erfolgt. Vorteilhaft ist eine Temperatur, die ebenfalls die Benutzung des sterilisierten Guts gestattet. Durch drücken der Verschlusswippe, das heißt durch einen wesentlichen Kraftaufwand, wird das wiedereinrückbare innenseitige Stellelement aus seiner zweiten Position in die erste Position wieder eingerückt und somit das sterile Gut freigegeben. Der Behälter kann nun nach Verwendung des Sterilguts erneut mit zu sterilisierendem Gut befüllt werden und der Verschluss ist erneut einsetzbar. Somit wird durch die erfindungsgemäße Lehre ein lang bestehendes Problem des Stands der Technik gelöst und eine Vorrichtung zur Verfügung gestellt, die wesentliche Arbeitsschritte des täglichen Laboralltags nicht nur einfacher, sondern auch sicherer gestaltet. Außerdem ist die Vorrichtung wiederverwendbar und erleidet durch die häufige Verwendung keine Funktionseinbußen.

Wesentliche Vorteile der Erfindung sind weiterhin, dass die Erfindung kostengünstig ist und flexibel an unterschiedliche Behälter und Behältergrößen angepasst werden kann.

Bevorzugt ist, dass der Thermoschalter ein Bimetall, bestehend aus zwei Schichten unterschiedlicher Materialien, ist und die Materialien stoffschlüssig und/oder formflüssig miteinander verbunden sind. Das Bimetall besteht vorteilhafterweise aus zwei Metallen, die sich bei Temperaturveränderungen unterschiedlich ausdehnen und aufgrund des festen Verbundes zu einer Krümmung des Bimetalls führen. Ursache für die Krümmung oder Verbiegung sind unterschiedliche stoffspezifische Eigenschaften der Metalle, wie beispielsweise unterschiedliche Wärmeausdehnungskoeffizienten beziehungsweise Längenausdehnungskoeffizienten. Somit kommt es bei einer Erwärmung zu einer Ausdehnung der Metalle um unterschiedliche Strecken und somit um eine Verbiegung des Bimetalles. Metalle die zur Herstellung des Bimetalles, beziehungsweise des Thermoschalters verwendet werden können, umfassen beispielsweise Zink und Stahl, Stahl in Kombination mit der Legierung Messing und/oder Eisen. Vorteilhafterweise werden die beiden Metalle stoffschlüssig und/oder formschlüssig miteinander verbunden, wodurch eine nichtlösbare Verbindung entsteht. Die Verbindungen umfassen kleben, schweißen und/oder löten. Die Verbindungen lassen sich insbesondere nur durch Zerstörung der Verbindungsmittel trennen. Es kann ebenfalls bevorzugt sein, die Metalle mittels einer Pressung zusammen zu fügen.

Vorteilhafterweise kann das Bimetall derart ausgewählt sein, dass bei einer spezifischen Temperatur eine Verformung des Bimetalls erfolgt. Somit können durch einfache materialspezifische Modifikation des Bimetalles, die Eigenschaften des Verschlusses geändert werden. Vorteilhafterweise kann ein Bimetall in den Verschluss integriert werden, bei dem abhängig von der Verwendung des Verschlusses bei einer hohen oder niedrigen Temperatur eine Verformung eintritt. So kann beispielsweise bei einer Sterilisationstechnik mit einer niedrigeren Temperatur, ein Thermoschalter aus einem Bimetall verwendet werden, welches sich schon bei dieser Temperatur verformt. Somit wird eine korrekte Funktionsweise des Verschlusses durch einen einfachen Austausch des Thermoschalters gewährleistet. Weiterhin ist vorteilhaft, dass die Herstellung des Thermoschalter, beziehungsweise des Bimetalls kostengünstig ist und auch die Größe des Schalters an die Größe des Verschlusses und an die Größe des Deckels und des Behälters angepasst werden kann. Vorteilhaft sind Bimetalle die auch bei den hohen Temperaturen der Sterilisation keine Funktionseinbußen aufzeigen und auch nicht korridieren. Zusätzlich kann der mechanische Effekt den das Bimetall, beziehungsweise der Thermoschalter auf das innenseitige wiedereinrückbare Stellelement ausübt verändert werden, da die Verbiegung des Bimetalles von den materialspezifischen Eigenschaften der Metalle abhängig ist und auch eine größere Bewegung des Bimetalles durch längere Metalle erreicht wird.

Es ist weiterhin bevorzugt, dass die Halterung des Thermoschalters aus Kunststoff und/oder Metall gefertigt ist. Kunststoffe bezeichnen Materialien, deren wesentliche Bestandteile aus solchen makromolekularen organischen Verbindungen bestehen, die synthetisch oder durch Abwandlung von Naturprodukten entstehen. Sie sind in vielen Fällen unter bestimmten Bedingungen (Wärme und Druck) schmelz- und formbar. Bevorzugte Kunststoffe umfassen Polyethylen, Polypropylen, Polyvenylchlorid, Polystirol, Polyamid, Polycarbonat, Silicone, Polyvenyl, Dyral und/oder Plytetrafluorethylen.

Metalle bezeichnen chemische Elemente, die sich im Gegensatz zu den Nichtmetallen im Periodensystem links der diagonalen Trennungslinie beginnend mit dem Element Berilium (zweite Gruppe) bis hin zum Pollonium (16. Gruppe) befinden, sowie deren Legierungen und intermetallische Verbindungen. Bevorzugte Metalle umfassen Aluminium, Eisen, Kupfer, Nickel, Zink und/oder Zinn oder Kombinationen hieraus. Vorteilhafterweise kann Stahl und/oder Edelstahl verwendet werden. Für nichtmetallische Materialien können in einer bevorzugten Ausführungsform Polymere verwendet werden. Polymere bezeichnen eine Substanz, die sich aus einem Kollektiv chemisch einheitlich aufgebauter, sich in der Regel aber hinsichtlich Polymerisationsgrad, Molmasse und Kettenlänge unterscheidender Makromoleküle (Polymermoleküle) zusammensetzt. Bevorzugt umfassen Polymere anorganische Polymere, metallorganische Polymere, voll- oder teilaromatische Polymere, Homopolymere, Grobpolymere, Biopolymere, chemisch modifizierte Polymere und/oder synthetische Polymere.

Die Fertigung der Halterung des Thermoschalters aus Kunststoff, beziehungsweise Polymeren weist eine hohe Beständigkeit gegen Korrosion und Verrottung auf und besitzt ein niedriges spezifisches Gewicht. Weiterhin kann die Halterung aus Kunststoff einfach an die Form des Thermoschalters angepasst werden, da der Kunststoff flexibel anpassbar und bearbeitbar ist. Das mechanische Verhalten des Kunststoffes in der Wärme kann durch Verfeinern des Kristallinenaufbaus der Komponenten erreicht werden, wodurch die Formbeständigkeit bei hohen Temperaturen, sowie die Wärmestandfestigkeit verbessert wird. Auch die Verwendung von Metallen zur Herstellung des Halters für den Thermoschalter ist vorteilhaft, da die Metalle eine hohe Wärmebeständigkeit aufweisen und durch die Verwendung von Legierungen eine nahezu innerte Oberfläche entsteht, die auch während des Sterilisationsprozesses, durch gegebenenfalls austretendes Sterilgut nicht beschädigt wird. Der Thermoschalter kann bevorzugt mittels Befestigungsmitteln an der Halterung oder dem Deckel befestigt werden. Hierbei umfassen Befestigungsmittel insbesondere Schrauben, Niete und Noppen. Jedoch kann es auch vorteilhaft sein, dass der Thermoschalter mittels stoffschlüssigen oder formschlüssigen Verbindungen an der Halterung befestigt wird und so ein nicht lösbarer Verbund besteht, der die Beständigkeit des Verschlusses verbessert.

Bevorzugt ist, dass das wiedereinrückbare innenseitige Stellelement aus Kunststoff und/oder Metall gefertigt ist und eine Nut aufweist. Die Fertigung des innenseitigen Stellelements aus Kunststoff erniedrigt vorteilhafterweise das Gesamtgewicht des Verschlusses und reduziert weiterhin die Herstellungskosten des Verschlusses. Das Stellelement aus Kunststoff kann optimal an die Größe des Veschlusses, beziehungsweise des Deckels angepasst werden, da der Kunststoff formbar ist und auch nach Fertigstellung des Stellelementes Modifikationen möglich sind. In einer weiteren Ausführungsform ist das Stellelement aus Metall gefertigt, wodurch eine hohe Wärmebeständigkeit des Stellelementes sichergestellt ist. Durch die bevorzugte Ausführungsform ist ein konstanter Betrieb des Verschlusses, beziehungsweise des Stellelementes sichergestellt, wobei auch durch die Bewegung des Stellelementes keine Abnutzung oder Beschädigung des Stellelementes erfolgen. Im Sinne der Erfindung beschreibt eine Nut eine längliche Vertiefung, die eine Innenform des wiedereinrückbaren innenseitigen Stellelementes ist. Ist der Verschluss in einer offenen Position, das heißt in der ersten Position, ist der Thermoschalter und das wiedereinrückbare innenseitige Stellelement derart zueinander angeordnet, dass durch eine Verformung des Thermoschalters eine Bewegung des Stellelementes in die zweite Position (geschlossen) erfolgt. Aus der zweiten Position kann das Stellelement, welches in der zweiten Position an der Verschlusswippe anliegt und diese in den Anschlag drückt, durch einen wesentlichen Kraftaufwand, der von außen auf die Verschlusswippe wirkt, in die erste Position gebracht werden. Vorteilhafterweise arretiert das innenseitige wiedereinrückbare Stellelement in dieser ersten Position, indem die Nut sich mit einem Halteelement verbindet und so eine Steckverbindung entsteht, die das Stellelement in der ersten Position hält. Das Halteelement kann vorteilhafterweise eine Feder umfassen. Die Steckverbindung beschreibt eine formschlüssige Verbindung und entsteht durch das Ineinandergreifen von mindesten zwei Verbindungspartnern (beispielsweise Nut und Feder), wodurch eine lösbare Verbindung generiert wird. Hierbei sind die Verbindungspartner nicht ohne Kraftübertragung voneinander lösbar. Es kann auch vorteilhaft sein, wenn die Verbindung zwischen Stellelement und Halteelement beispielsweise über eine Schnappfederverbindung entsteht. Das Halten des Stellelementes in der ersten Position kann jedoch auch ohne Nut erfolgen, indem das Stellelement in der ersten Position derart arretiert wird, dass das Stellelement nur durch einen einwirkenden Druck und nicht selbstständig aus dieser Position in die zweite Position gelangen kann. Durch die bevorzugte Ausführungsform ist ein dauerhafter Betrieb des Verschlusses ermöglicht, wobei auch die Verwendung des Verschlusses bei hohem Druck und hohen Temperaturen ohne Funktionsverluste garantiert ist.

Es ist bevorzugt, dass das innenseitige wiedereinrückbare Stellelement an einer Achse federelastisch gelagert ist. Durch eine Halterung wird das Stellelement in einer ersten Position gehalten und bei einer Erwärmung des Verschlusses, beziehungsweise bei einer Sterilisation des Behälters wird der Thermoschalter verformt und das innenseitige Stellelement in eine zweite Position bewegt. Hierbei übt die Verformung des Thermoschalters kurzzeitig einen Druck - eine Kraft - auf das Stellelement aus, wodurch die Verbindung zwischen Stellelement und Halteelement gelöst wird und sich das Stellelement durch die federelastische Lagerung in die zweite Position bewegt. In der zweiten Position übt es, ebenfalls bedingt durch die federelastische Lagerung, einen Druck auf die Verschlusswippe aus, durch welchen diese in einen Anschlag gedrückt wird.

Im Sinne der Erfindung beschreibt eine elastische Feder ein Element, welches sich unter Belastung gezielt verformt und bei Entlastung wieder in die ursprüngliche Gestalt zurückkehrt. Dabei wird potentielle Energie gespeichert, die bei der Rückfederung unter Berücksichtigung der Reibungsverluste in Form von Arbeit wieder abgegeben werden kann. Hierbei kann beispielweise ein aus Draht gefertigtes, schrauben- oder spiralförmiges, gewickeltes oder stabförmiges, gestrecktes oder abgeknicktes Element verwendet werden.

Bevorzugt ist, wenn das Stellelement an einem Ende federelastisch in einer Achse gelagert ist. Hierbei kann die Achse aus Kunststoff und/oder Metall gefertigt sein. Durch die vorteilhafte Ausführungsform wird das Gewicht des Verschlusses reduziert und sichergestellt, dass auch bei einem Dauerbetrieb des Stellelementes, keine Minderung der Funktionsfähigkeit erfolgt. Durch eine Anpassung der federelastischen Lagerung des Stellelementes kann der Druck, den das Stellelement auf die Verschlusswippe ausübt variiert werden, indem beispielsweise eine stärkere Feder verbaut wird. Es kann beispielsweise auch vorteilhaft sein, wenn das Stellelement mittig an einer Achse befestigt ist, wobei eine Seite des Stellelementes in räumlicher Nähe zu dem Thermoschalter angeordnet ist und das zweite entgegengesetzte Ende des Stellelementes federelastisch gelagert ist.

Eine weitere bevorzugte Ausführungsform ist, dass das wiedereinrückbare innenseitige Stellelement ein signalerzeugendes Element aufweist. In der offenen Position des Verschlusses befindet sich das Stellelement in räumlicher Nähe zu dem Thermoelement und die Verschlusswippe ist durch einen geringen Kraftaufwand eindrückbar, wodurch der Behälter geöffnet werden kann. Während der Sterilisation verformt sich der Thermoschalter und bewirkt eine Bewegung des Stellelementes in eine zweite Position in der das Stellelement auf die Verschlusswippe wirkt und diese in einen Anschlag drückt. Vorteilhafterweise ist ein signalerzeugendes Element an dem Stellelement befestigt, wodurch sichtbar angezeigt wird, in welcher Position sich das Stellelement befindet und folglich der Verschluss offen oder geschlossen ist. Ein signalerzeugendes Element umfasst beispielsweise einen Farbumschlag oder eine Bewegung eines Anzeigeelementes, wodurch die Position des Stellelementes von außerhalb des Verschlusses oder des Deckels erkennbar ist. So kann der Benutzer des Sterilgutes leicht erkennen, ob der Behälter bereits geöffnet wurde oder nicht, ohne die Verschlusswippe zu betätigen. Es kann auch vorteilhaft sein, dass das signalerzeugende Element an einem anderen Bestandteil des Verschlusses, wie beispielsweise der Verschlusswippe oder der Feder befestigt wird. Durch die bevorzugte Ausführungsform kann einfach und schnell erkannt werden, ob der Behälter bereits nach der Sterilisation geöffnet wurde und dementsprechend das Sterilgut keine Sterilität mehr aufweist, ohne den Verschluss des Behälters betätigen zu müssen. Des Weiteren ermöglicht die bevorzugte Ausführungsform eine einfache Verwaltung von sterilisiertem Gut oder Behältern, da diese entsprechend ihrer signalerzeugenden Elemente (offen oder geschlossen) sortiert werden können. Hierdurch kann eine effiziente Arbeitsweise mit einer hohen Qualität ermöglicht werden.

Es ist bevorzugt, dass die Verschlusswippe aus Kunststoff und/oder Metall gefertigt ist. Die Verschlusswippe wird nach Sterilisation durch das Stellelement in einen Anschlag gedrückt und in einer Position arretiert, in der die Verschlusswippen nur durch einen wesentlichen Kraftaufwand von außen eindrückbar ist. In dieser Position ist der Behälter verschlossen. Vorteilhafterweise ist die Verschlusswippe an einer Achse befestigt, so dass die Verschlusswippe eine lösbare Verbindung mit dem Behälter, der durch den Deckel bedeckt wird, herstellt. Durch einen Druck auf die Verschlusswippe wird diese Verbindung gelöst und der Deckel ist von dem Behälter entfernbar. Hierbei kann es sich beispielsweise um eine Schnappverbindung handeln. Vorteilhafterweise ist die Verschlusswippe aus Kunststoff oder Leichtmetall gefertigt, wodurch ein geringes Gewicht des Verschlusses garantiert ist und keine Verformung der Verschlusswippe bei hohem Druck oder hohen Temperaturen erfolgt. Die Form der Verschlusswippe kann einfach an den Verschluss, beziehungsweise an den Deckel angepasst werden, wodurch eine flexible Verwendung des Verschlusses, beziehungsweise des Behälter ermöglicht wird.

Ein weiterer Aspekt der Erfindung ist, dass der Verschluss und/oder der Deckel ein Kunststoffkissen, umfassend eine Salzlösung und ein Metallplättchen aufweist. Während der Sterilisation wird die in dem Kunststoffkissen vorhandene Lösung verflüssigt und dieser Zustand wird auch bei Abkühlung des Verschlusses, beziehungsweise des sterilisierten Behälters und des Deckels beibehalten. Das Kunststoffkissen ist derart in den Verschluss und/oder den Deckel eingearbeitet, dass die Lösung, beziehungsweise das Kunststoffkissen, von außen sichtbar ist. Somit ist erkennbar ob eine Sterilisation des Behälters erfolgte, da durch die Sterilisation des Behälters die in dem Kunststoffkissen vorhandene Lösung transparent und flüssig ist. Durch eine Krafteinwirkung auf die Verschlusswippe wird beispielsweise das Stellelement in eine erste Position bewegt und außerdem wird durch eine mechanische Bewegung das in dem Kunststoffkissen vorhandenen Metallplättchen mit Druck belastet. Durch den auf das Metallplättchen ausgeübten Druck, der beispielweise auch von einem anderen Element des Verschlusse und/oder des Deckels ausgeübt werden kann, wird die Kristallisation der in dem Kunststoffkissen vorhandenen Salzlösung ausgelöst. Der Druck bewirkt die Freisetzung von Kristallisationskeimen, die wiederum eine vollständige Kristallisation der übersättigten Lösung initiieren. Es findet ein Phasenübergang von flüssig nach fest statt und durch die reversible thermodynamische Zustandsänderung wird Wärme freigegeben. Die Zustandsänderung, beziehungsweise die kristalline Lösung ist von außen gut sichtbar und dient dementsprechend als Indikator für das erfolgte Öffnen des Verschlusses und/oder des Deckels. Hierdurch wird verhindert, dass ein bereits nach der Sterilisation geöffneter Behälter und ein gegebenenfalls bereits kontaminiertes Sterilgut erneut verwendet wird. Die in dem Kunststoffkissen vorliegende kristalline Lösung kann durch erneute Sterilisation des Behälters, beziehungsweise des Verschlusses und/oder des Deckels, erneut in eine transparente flüssige Lösung überführt werden. Auch durch diesen Aspekt der Erfindung wird eine Vorrichtung bereitgestellt, die eine Beurteilung darüber ermöglicht, ob eine Sterilisation stattgefunden hat und ob das Behältnis, welches das zu sterilisierende Gut enthält, bereits nach der Sterilisation geöffnet wurde. Überraschenderweise stellt das in den Verschluss und/oder Deckel integrierte Kunststoffkissen eine effiziente Lösung dar, die einem Benutzer des sterilisierten Behälters einfach dargestellt, ob eine Sterilisation erfolgte und ob der Behälter nach der Sterilisation bereits geöffnet wurde.

Es ist bevorzugt, dass die Salzlösung Natriumacetat-Trihydrat ist. Das bevorzugte Salzhydrat wird bei der Temperatur der Sterilisation verflüssigt und behält diesen Zustand bis weit unter den Nullpunkt bei. In dem flüssigen Zustand liegt das Salz in einem metastabilen Zustand vor, das heißt das Salz ist stabil gegenüber kleineren Änderungen, jedoch instabil gegenüber großen Änderungen, die beispielsweise durch einen erhöhten Druck auf das Kunststoffkissen ausgelöst werden können. Bevorzugt liegt das Natriumacetat-Trihydrat in einer übersättigten Lösung in dem Kunststoffkissen vor. Wird nun auf das in einem flüssigen Zustand vorliegende Natriumacetat-Trihydrat ein Druck ausgeübt, durch beispielsweise drücken eines Metallplättchens, kommt es zur Bildung von Kristallisationskeimen, die Kristallstrukturen ausbilden und ein lawinenartiges Kristallwachstum bewirken. Es kann weiterhin vorteilhaft sein, dass Natriumsulfat sowie alaune Salze verwendet werden. Die bevorzugte Ausführungsform ermöglicht eine einfache und schnelle Beurteilung darüber, ob der Behälter bereits geöffnet wurde ob eine Sterilisation erfolgte, da die in den Verschluss integriert Salzlösung durch die Sterilisation (einwirkende Temperatur) in einen flüssigen Zustand gebracht wird und dieser Zustand der Salzlösung sichtbar in dem Verschluss, beziehungsweise im Deckel erkennbar ist. Nach Öffnen des Verschlusses, beziehungsweise nach Öffnen des Behälters wird die Lösung duch eine Aggratzustandsänderung in einen kristallinen Zustand befördert. Somit ist leicht erkennbar ob der Behälter nach der Sterilisation bereits geöffnet wurde oder nicht. Vorteilhafterweise ist das Kunststoffkissen und die darin befindliche Lösung wiederverwendbar und stellt somit einen kostengünstigen Indikator für Sterilisationsprozesse dar.

Die Erfindung betrifft weiterhin ein Verfahren zum Verschließen eines Verschlusses, umfassend einen Thermoschalter, ein innenseitiges wiedereinrückbares Stellelement und eine Verschlusswippe, wobei der Thermoschalter in einer Halterung befestigt ist und das wiedereinrückbare innenseitige Stellelement an einem Ende federelastisch gelagert ist und mit seinem zweiten Ende derart angeordnet ist, dass eine temperaturbedingte Formveränderung des Thermoschalters, einen Druck auf das Stellelement ausübt und sich das innenseitige Stellelement aus einer ersten Position in eine zweite Position bewegt, und das innenseitige Stellelement in der zweiten Position die Verschlusswippe in einen Anschlag drückt. Vorteilhafterweise ist der Thermoschalter als Bimetall ausgestalltet. Durch die Temperatureinwirkung der Sterilisation auf den Verschluss und somit den Thermoschalter, wird eine Verformung des Schalters bewirkt, die einen Druck auf das in der räumlichen Nähe des Thermoschalters angeordneten Stellelementes ausübt. Die Verformung ist reversibel und wird durch eine Temperaturerniedrigung katalysiert, wodurch der Schalter wieder in seine ursprüngliche Form zurückkehrt.

Das Stellelement weist vorteilhafterweise eine Nut auf, die vorteilhafterweise in der ersten Position mit einem Halteelement verbunden ist und somit das Stellelement in der ersten Position arretiert. Hier können weitere formschlüssige Verbindungsarten verwendet werden, die das Stellelement in der ersten Position halten.

Eine bevorzugte Ausführungsform ist ein Stellelement aus Kunststoff und/oder Metall. Durch den Druck des verformten Thermoschalters wird die Verbindung zwischen dem Halteelement und dem Stellelement gelöst und das Stellelement bewegt sich in eine zweite Position, in der es die Verschlusswippe in einen Anschlag drückt. Der Verschluss des Deckels ist in einer offenen Position, wenn das Stellelement in der ersten Position angeordnet ist und in einer geschlossenen Position, wenn das Stellelement in der zweiten Position an der Verschlusswippe anliegt. Der Verschluss, beziehungsweise die Verschlusswippe ist in der zweiten Position nur durch einen wesentlichen Kraftaufwand von außen eindrückbar. Wird die notwendige Kraft aufgewendet, wird das Stellelement mittels Kraft in seine erste Position befördert, wobei erneut eine lösbare Verbindung mit dem Halteelement hergestellt wird. Somit wird dem Benutzer einfach dargestellt, ob der Behälter nach der Sterilisation bereits geöffnet wurde, da der Behälter durch einen geringen Kraftaufwand zu öffnen ist, wenn das Stellelement in der ersten Position arretiert ist. Vorteilhafterweise kann der Deckel des Behälters in der offenen Position einfach geöffnet werden, was jedoch in der geschlossenen Position nicht möglich ist.

Die Erfindung betrifft auch die Verwendung eines Thermoschalters zum Verschließen eines Verschlusses, eines Sterilgutbhälters, wobei eine Formänderung des Thermoschalters eine Bewegung eines innenseitigen wiedereinrückbaren Stellelement von einer ersten Position in eine zweite Position bewirkt und das Stellelement in der zweiten Position an einer Verschlusswippe anliegt und diese in einen Anschlag drückt.
Im Sinne der Erfindung ist ein Thermoschalter oder Temperaturschalter ein Schalter, dessen Schaltzustand sich in Abhängigkeit von der Temperatur ändert. Bevorzugt ist die Verwendung eines Bimetalles als Thermoschalter. Durch eine materialspezifische Temperaturabhängigkeit des Schalters wird eine Verformung erreicht, da bei einer Erwärmung des Thermoschalters sich die Metalle um unterschiedliche Strecken ausdehnen und eine Verbiegung des Bimetalles bewirken.

In der räumlichen Nähe des Thermoschalters ist das Stellelement angeordnet. Das Stellelement wird durch die termperaturabhängige Verformung des Thermoschalters bewegt, da die Verformung einen Druck auf das Stellelement ausübt und dieses vorteilhafterweise aus einer Schnappverbindung oder einer sonstigen formschlüssigen Verbindung mit einem Haltelement gedrückt wird. Das Stellelement ist vorteilhafterweise federelastisch gelagert und bewegt sich von einer ersten Position in eine zweite Position. In der zweiten Position wird vorteilhafterweise durch die federelastische Wirkung ein Druck auf die Verschlusswippe ausgeübt und diese in einen Anschlag gedrückt. In dieser Position ist der Verschluss, beziehungsweise die Verschlusswippe nur durch einen wesentlichen Kraftaufwand eindrückbar und der Behälter ist verschlossen. Nach Öffnen des Behälters, wird durch die von außen auf die Verschlusswippe wirkende Kraft das Stellelement aus der zweiten Position in die erste Position bewegt, wo es vorteilhafterweise mit dem Halteelement interagiert und von diesem in der ersten Position arretiert. Es befindet sich in räumlicher Nähe zu dem Thermoschalter und zwar derart, dass durch eine Verformung des Thermoschalters ein Druck auf das Stellelement ausgeübt wird. Nach Abkühlung des Behälters beziehungsweise des Verschlusses kehrt der Thermoschalter in seine ursprüngliche Form zurück und übt keinen Druck auf das Stellelement aus. Durch die Verwendung eines Thermoschalters in einem Verschluss eines Sterilgutbehälters wird dem Benutzer des Behälters zuverlässig angezeigt, ob der Behälter bereits nach der Sterilisation geöffnet wurde und das Sterilgut gegebenenfalls keine Sterilität mehr aufweist. Die Arbeit in Laboren wird so sicherer und effizienter. Außerdem ist der Verschluss einfach und kostengünstig an unterschiedliche Größen anpassbar, das heißt der Verschluss kann an die Größe des Behälters einfach angepasst werden. Zusätzlich kann beispielsweise an dem Stellelement oder an einem anderen Bauteil des Verschlusses ein signalerzeugendes Element angeordnet werden, was abhängig von der Position des Stellelementes dem Benutzer von außen anzeigt, ob der Behälter bereits nach der Sterilisation geöffnet wurde. Hierbei kann es sich um ein sich bewegendes Element, wie beispielsweise ein Fähnchen handeln oder um eine farbliche Anzeige, wodurch die Arbeit mit Sterilgutbehältern erheblich vereinfacht wird.

Weitere vorteilhafte Maßnahmen sind in den Unteransprüchen beschrieben.

Die Erfindung wird nunmehr anhand von Figuren beispielhaft beschrieben, ohne auf die Beispiele begrenzt zu sein. Es zeigen:
- Figur 1: Seitenansicht eines Sterilgut-Behälters mit einem geschlossenen Verschluss
- Figur 2: Detailansicht von einem geschlossenen Verschluss
- Figur 3: Detailansicht von einem Übergangszustand beim Öffnen des Verschlusses
- Figur 4: Detailansicht von einem geöffneten Verschluss
- Figur 5: Detailansicht von einem Übergangszustand beim Schließen des Verschlusses

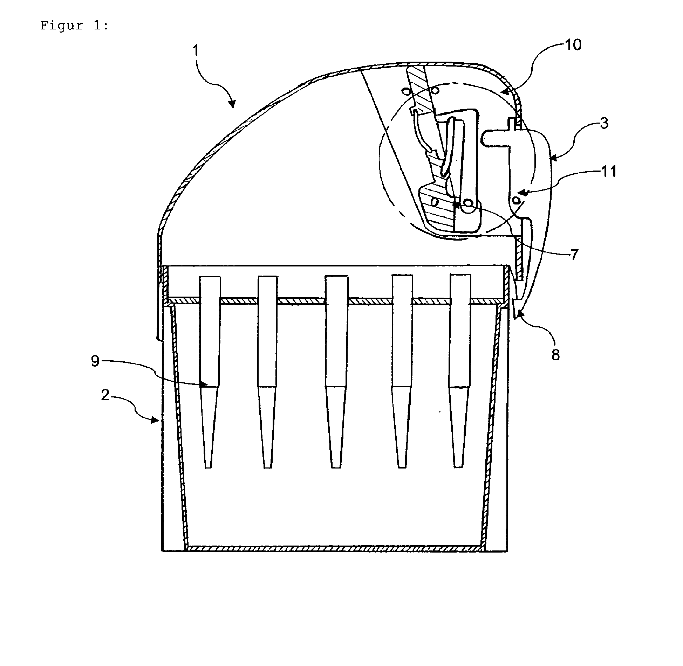

Die Figur 1 zeigt eine Seitenansicht eines Sterilgut-Behälters mit einem geschlossenen Verschluss. Auf einem Sterilgut-Behälter 2, ist ein Deckel 1 mit einem Verschluss 10 aufgebracht. Der Deckel 1 kann beispielsweise mittels einer Schnappverbindung oder einer sonstigen dem Fachmann bekannten Verbindung 8 mit dem Deckel verbunden sein. Es ist bevorzugt, dass der Deckel 1 auf einer Seite anhebbar ist, das heißt der Deckel 1 ist auf wenigstens einer Seite über eine reversible Verbindung 8 mit dem Behälter 2 verbunden. Die Verbindung 8 wird bevorzugt über die Verschlusswippe 3 hergestellt. Hierfür kann beispielsweise die Verschlusswippe 3 mit dem Behälter 2 über eine Schnappverbindung verbunden sein. Die Verschlusswippe 3 ist vorteilhafterweise über eine Achse 11 beweglich gelagert. Durch einen auf die Verschlusswippe 3 ausgeübten Druck, kann die Verbindung 8 zwischen Deckel 1 und Behälter 2 gelöst werden und der Deckel 1 ist von dem Behälter 2 anhebbar. In einem offenen Zustand kann der Behälter 2 mit Sterilgut 9 befüllt werden oder dieses nach Sterilisation aus dem Behälter 2 entnommen werden. In einem geschlossenen Zustand, vorteilhafterweise nach einer Sterilisation, befindet sich das innenliegende Stellelement 4 in einer räumlichen Nähe zu der Verschlusswippe 3, so dass diese nur begrenzt bewegbar ist. Bevorzugt wird die Verschlusswippe 3 durch das Stellelement 4 in den Anschlag gedrückt, das heißt die Verschlusswippe kann nicht mehr bewegt werden und der Deckel 1 ist nicht von dem Behälter 2 anhebbar. Es kann jedoch auch bevorzugt sein, dass das Stellelement 4 die Verschlusswippe 3 nicht in den Anschlag drückt, sondern nur die Bewegungsfreiheit der Verschlusswippe 3 einschränkt und die Verbindung 8 zwischen Verschlusswippe 3 und Behälter 2 nicht trennbar ist. Um die Bewegungsfreiheit der Verschlusswippe 3 zu erhöhen und den Deckel 1 von dem Behälter 2 anzuheben, muss ein wesentlicher Kraftaufwand, das heißt ein Druck auf die Verschlusswippe 3 ausgeübt werden.

Das Stellelement 4 ist vorteilhafterweise mit einem Haltelement 6 verbunden. Das Stellelement 4 sowie der Thermoschalter 5 sind bevorzugt in einer Halterung 7 gelagert. Bevorzugt ist ebenfalls, dass der Thermoschalter 5 bedingt durch die Halterung 7 nur in einer Ebene bewegbar ist.

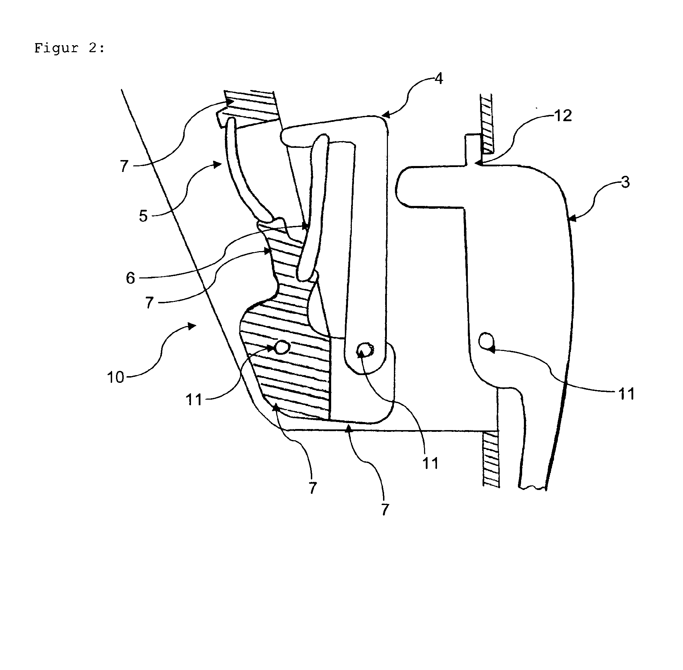

Die Figuren 2 bis 5 zeigen Detailansichten von dem Verschluss in verschiedenen Zuständen. Die Figur 2 zeigt den Verschluss in geschlossenem Zustand. Durch die Temperatureinwirkung der Sterilisation ändert der Thermoschalter 5 seine Form und bewegt das Stellelement 4 in eine zweite Position. Hierbei kann es vorteilhaft sein, wenn die Bewegung des Stellelementes 4 durch eine Feder unterstütz wird. Die Formänderung des Thermoschalters 5 bewegt das Stellelemente 4 aus der arretierten ersten Position und durch das Lösen der Arretierung kann beispielsweise eine Entspannung der Feder das Stellelement 4 in die zweite Position befördern. In dieser Position drückt das Stellelement 4 bevorzugt auf die Verschlusswippe 3, so dass die Verschlusswippe 3 von außen nicht durch einen geringen Kraftaufwand eindrückbar ist. Der Behälter ist geschlossen. Somit kann nach erfolgreicher Sterilisation, das heißt nach Formänderung des Thermoschalters 5 der Behälter nicht ohne wesentlichen Kraftaufwand geöffnet werden.

Vorteilhafterweise ist der Thermoschalter 5 in einer Halterung 7 gelagert, so dass eine Bewegung des Thermoschalters 5 bevorzugt nur in einer Ebene erfolgen kann. Der Thermoschalter 5 ist vorteilhafterweise aus einem Bimetall gefertigt, das aus zwei Metallen besteht, die miteinander stoffschlüssig oder formschlüssig verbunden sind. Das Bimetall ändert durch eine Temperatureinwirkung seine Form. Das Stellelement 4 ist bevorzugt beweglich über eine Achse 11 gelagert.

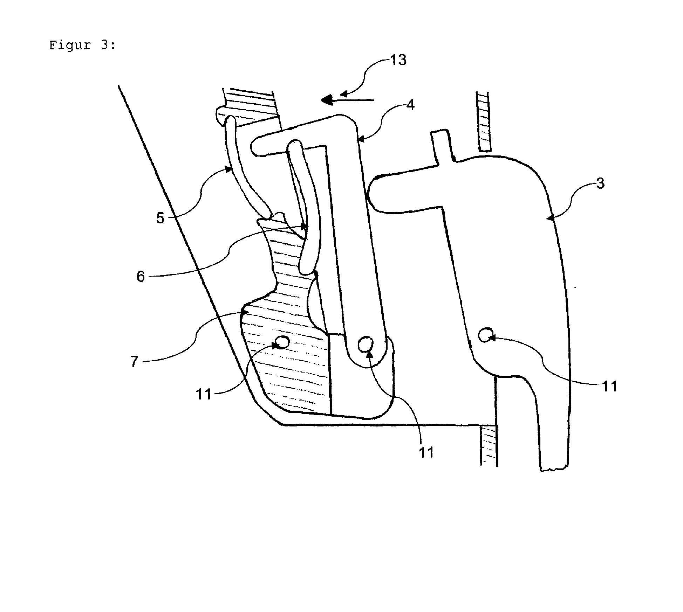

Wie aus Figur 3 ersichtlich wird, kann durch einen wesentlichen Kraftaufwand die Verschlusswippe 3 eingedrückt werden, um so die Verbindung zwischen dem Deckel und dem Behälter zu unterbrechen. Hierdurch kann der Deckel angehoben werden und beispielsweise das Sterilgut aus dem Behälter entfernt werden. Gleichzeitig wird durch das Eindrücken die Verschlusswippe 3 auf das Stellelement 4 gedrückt, wodurch das Stellelement 4 in die erste Position bewegt wird. Vorteilhafterweise wird durch hierdurch eine Feder gespannt, das heißt aus einem entspannten in einen gespannten Zustand überführt. Die Bewegung des Stellelementes 4 wird durch den Pfeil 13 angezeigt. Vorteilhafterweise arretiert das Stellelement 4 in der ersten Position. Hierfür kann beispielsweise eine Schnappverbindung genutzt werden, die über eine Nut-Feder-Verbindung das Stellelement 4 mit einem Halteelement 6 verbindet und das Stellelement 4 in der ersten Position hält. Vorteilhafterweise ist ein Geräusch zu hören, wenn das Stellelement 4 in der ersten Position arretiert. Dieses Geräusch kann für einen Benutzer ein Signal darstellen, dass der Verschluss, beziehungsweise der Behälter aus einem geschlossenen in einen geöffneten Zustand überführt wurde. Vorteilhafterweise kann ebenfalls ein signalerzeugendes Element, beispielsweise ein optischer Signalgeber dem Benutzer signalisieren, in welchem Zustand der Behälter gerade ist. Der Signalgeber kann bevorzugt auf der Oberfläche des Behälters angebracht sein, so dass es für einen Benutzer sichtbar ist. Vorteilhafterweise ist der Signalgeber mit einem Bestandteil des Verschlusses verbunden, beispielsweise dem Stellelement. Durch eine Bewegung des Stellelementes erfolgt bevorzugt auch eine Bewegung des optischen Signalgebers, wodurch ein Benutzer schnell und einfach erkennen kann, ob der Behälter verschlossen oder geöffnet ist.

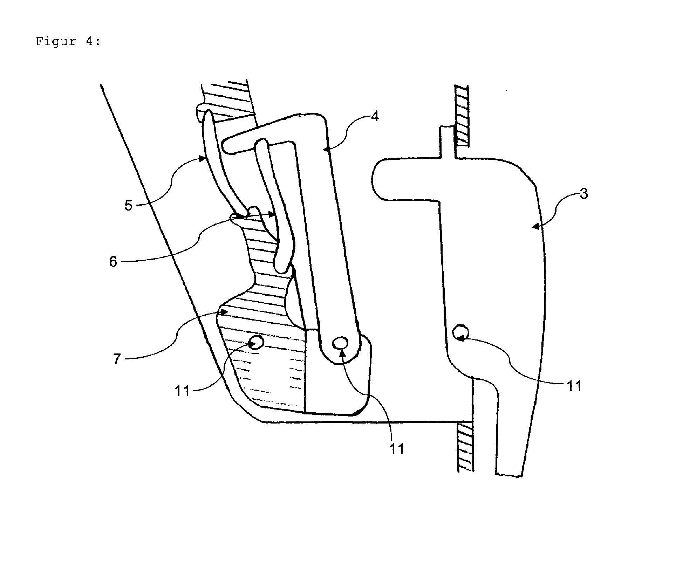

Die Figur 4 zeigt den Verschluss in der geöffneten Position. In der geöffneten Position des Verschlusses ist das Stellelement 4 in der ersten Position und in räumlicher Nähe zu dem Thermoschalter 5. In dem geöffneten Zustand des Verschlusses kann die Verschlusswippe 3 frei bewegt werden. Das heißt, die Verschlusswippe 3 kann von außen durch einen geringen Kraftaufwand eingedrückt werden und der Deckel ist anhebbar.

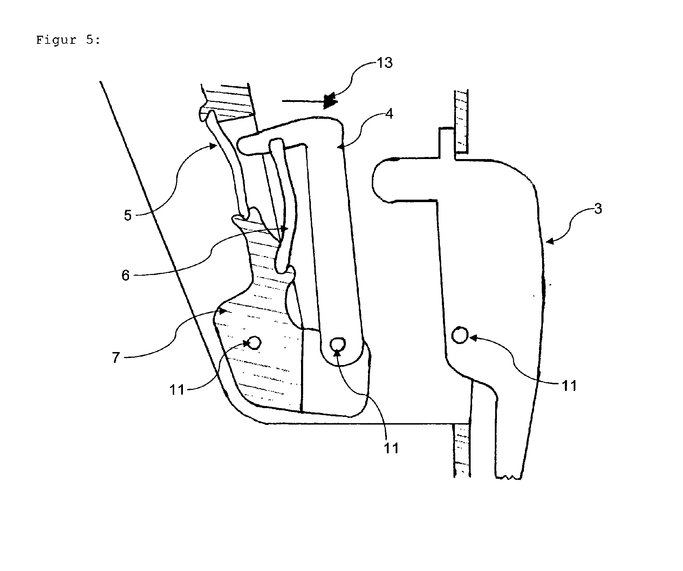

Die Figur 5 stellt schematisch den Übergangszustand während der Sterilisation dar. Durch die Temperatureinwirkung der Sterilisation erfolgt eine Verformung des Thermoschalters 5. Der Thermoschalter 5 besteht bevorzugt aus zwei Metallen, die miteinander verbunden sind und unterschiedliche Längenausdehnungskoeffizienten beziehungsweise Wärmeausdehnungskoeffizienten besitzen. Durch diese materialspezifischen Faktoren verformen sich die Metalle bei Erwärmung um unterschiedliche Strecken. Die unterschiedliche Verlängerung der Metalle führt wiederum zu einer Verbiegung des Bimetalles, das heißt zu einer Verformung des Thermoschalters 5. Bedingt durch die räumliche Nähe zwischen dem Thermoschalter 5 und dem Stellelement 4, wird durch die Verformung des Thermoschalters 5 ein Druck auf das Stellelement 4 ausgeübt. Der Druck wiederum bewirkt, dass das Stellelement 4 aus der ersten Position in die zweite Position bewegt wird, in der das Stellelement 4 bevorzugt auf die Verschlusswippe 3 drückt. Es kann jedoch auch bevorzugt sein, dass das Stellelement 4 nicht auf die Verschlusswippe 3 drückt. Hierdurch kann vorteilhafterweise die Bewegungsfreiheit der Verschlusswippe 3 eingeschränkt sein und die Verbindung zwischen der Verschlusswippe 3 und dem Behälter kann nicht ohne wesentlichen Kraftaufwand gelöst werden. Der Behälter ist verschlossen. Somit wird durch die Temperatureinwirkung der Sterilisation auf den Thermoschalter 5 eine Verformung des Thermoschalters 5 bewirkt, die den Verschluss in einen geschlossenen Zustand überführt. Die Verformung des Thermoschalters 5 ist vorteilhafterweise reversibel und der Thermoschalter 5 kehrt nach Erreichen einer spezifischen Temperatur, die bevorzugt niedriger als die Sterilisationstemperatur ist, wieder in seine ursprüngliche Form zurück. Eine Person, die das sterilisierte Sterilgut verwenden möchte, muss einen wesentlichen Kraftaufwand aufwenden, um die Verschlusswippe 3 einzudrücken, wodurch das Stellelement 4 in die erste Position befördert wird. Vorteilhafterweise kann das Stellelement 4 über eine Feder federelastisch gelagert sein, so dass eine Kraft aufgewendet werden muss, um das Stellelement 4 aus der zweiten Position in die erste Position zu bewegen. Durch die Aufwendung eines wesentlichen Kraftaufwand oder bevorzugt durch einen optischen Signalgeber kann einer Person signalisiert werden, ob der Verschluss in der offenen oder geschlossenen Position vorliegt. Es kann somit einfach und schnell angezeigt werden, ob das Sterilgut noch Sterilität aufweist, das heißt ob der Behälter nach Sterilisation bereits geöffnet wurde oder nicht.

### Bezugszeichenliste

- 1: Deckel
- 2: Sterilgut-Behälter
- 3: Verschlusswippe
- 4: Stellelement
- 5: Thermoschalter
- 6: Halteelement
- 7: Halterung
- 8: Verbindung
- 9: Sterilgut
- 10: Verschluss
- 11: Achse
- 12: Element
- 13: Pfeil

## Patentansprüche

1. Verschluss eines Behälters für Sterilgut, der mindestens zwei Positionen (offen/geschlossen) einnehmen kann, umfassend eine Verschlußwippe, ein innenseitiges wiedereinrückbares Stellelement und einen in einer Halterung ortsfest befestigten Thermoschalter,
**dadurch gekennzeichnet dass**
in der ersten Position (offen), der Thermoschalter und das wiedereinrückbare innenseitige Stellelement derart zueinander angeordnet sind, dass eine Verformung des Thermoschalters eine Bewegung des wiedereinrückbaren innenseitigem Stellelement in die zweite Position (geschlossen) bewirkt und in der zweiten Position, das wiedereinrückbare innenseitige Stellelement an der Verschlußwippe anliegt und diese in einen Anschlag drückt.

2. Vorrichtung nach Anspruch 1
**dadurch gekennzeichnet, dass**
der Thermoschalter ein Bimetall bestehend aus zwei Schichten unterschiedlicher Materialien ist und die Materialien stoffschlüssig und/oder formschlüssig miteinander verbunden sind.

3. Vorrichtung nach einem oder mehreren der vorherigen Ansprüche **dadurch gekennzeichnet, dass**
die Halterung des Thermoschalters aus Kunststoff und/oder Metall gefertigt ist.

4. Vorrichtung nach einem oder mehreren der vorherigen Ansprüchen,
**dadurch gekennzeichnet, dass**
das wiedereinrückbare innenseitige Stellelement aus Kunststoff und/oder Metall gefertigt ist und eine Nut aufweist.

5. Vorrichtung nach einem oder mehreren der vorherigen Ansprüchen,
**dadurch gekennzeichnet, dass**
das innenseitige wiedereinrückbare Stellelement auf einer Achse federelastisch gelagert ist.

6. Vorrichtung nach einem oder mehreren der vorherigen Ansprüchen,
**dadurch gekennzeichnet, dass**
das wiedereinrückbare innenseitige Stellelement ein signalerzeugendes Element aufweist.

7. Vorrichtung nach einem oder mehreren der vorherigen Ansprüchen,
**dadurch gekennzeichnet, dass**
das außenseitige wiedereinrückbare Stellelement aus Kunststoff und/oder Metall gefertigt ist.

8. Vorrichtung insbesondere nach einem der vorherigen Ansprüche, **dadurch gekennzeichnet, dass**
der Verschluss ein Kunststoffkissen umfassend eine Salzlösung und ein Metall-Plättchen, aufweist.

9. Vorrichtung nach Anspruch 10,
**dadurch gekennzeichnet, dass**
die Salzlösung bevorzugt Natriumacetat-Trihydrat ist.

10. Verfahren zum Verschließen eines Verschlusses, umfassend einen Thermoschalter, ein innenseitiges und ein außenseitiges wiedereinrückbares Stellelement, wobei der Thermoschalter in einer Halterung befestigt ist und das wiedereinrückbare innenseitige Stellelement an einem Ende federelastisch gelagert ist und mit seinem zweiten Ende derart angeordnet ist, dass
eine temperaturbedingte Formveränderung des Thermoschalters, einen Druck auf das Stellelement ausübt und sich das innenseitige Stellelement aus einer ersten Position in eine zweite Position bewegt, und
das innenseitige Stellelement in der zweiten Position das außenseitiges wiedereinrückbares Stellelement in einen Anschlag drückt.

11. Verwendung eines Thermoschalters zum Verschließen eines Verschlusses Sterilgutbehälters,
**dadurch gekennzeichnet, dass**
eine Formänderung der Thermoschalter eine Bewegung des innenseitigem wiedereinrückbarem Stellelement von einer ersten Position in eine zweite Position bewirkt und das Stellelement in der zweiten Position an Verschlusswippe anliegt und diese in einen Anschlag drückt.
